# EUROPÄISCHE PATENTANMELDUNG

(11) **EP 1 293 192 A2**
(43) Veröffentlichungstag der Anmeldung: **19.03.2003**
(21) Anmeldenummer: 02019394.2
(22) Anmeldetag: 30.08.2002
(51) Int. Cl.: A61K 7/13

(54) **Haarfärbemittel und Verfahren zum Färben von menschlichen Haaren**

(30) Priorität: 12.09.2001 DE 10144837; 17.09.2001 DE 10145755; 17.09.2001 DE 10145756; 17.09.2001 DE 10145754
(71) Anmelder: KPSS-Kao Professional Salon Services GmbH, 64297 Darmstadt (DE)
(72) Erfinder: Lorenz, Heribert, 64401 Gross-Bieberau (DE)

(57) **Zusammenfassung**

Eine Haarfärbemittelzusammensetzung auf Basis eines Oxidationsfarbstoffvorprodukt-Systems, enthaltend
a) als Oxidationsfarbstoffvorprodukte eine Kombination aus mindestens einem Tetraaminopyrimidin, insbesondere 2,4,5,6-Tetraaminopyrimidin, und/oder einem Hydroxytriaminopyrimidin, insbesondere 4-Hydroxy-2,5,6-triaminopyrimidin bzw. deren Salzen, und
b) mindestens eine Kupplersubstanz, ausgewählt aus Resorcin, 2-Methylresorcin und/oder 4-Chlorresorcin,

a) Dihydroxyaceton, Alloxan und Methylglyoxal, und
b) Kaliumjodid
ergibt nach dem Vermischen mit einer Peroxidzusammensetzung auf dem Haar ausdrucksvolle, intensive Rotfärbungen.

## Beschreibung

Die Erfindung betrifft ein Haarfärbemittel auf Basis von Oxidationsfarbstoff-Vorprodukten, das nach dem Vermischen mit einer Peroxid-Zusammensetzung auf das menschliche Haar aufgebracht wird, und ein Verfahren zur Haarfärbung unter Verwendung dieses Mittels.

Der die Oxidationsfarbstoffvorprodukte enthaltenden Zusammensetzung wird grundsätzlich ein Reduktionsmittel, vorzugsweise Ascorbinsäure oder ein Alkalisulfit, zugesetzt, um diese Vorprodukte gegen eine unerwünschte vorzeitige Oxidation zu stabilisieren (vgl. hierzu beispielsweise die Monographie von K. Schrader, Grundlagen und Rezepturen der Kosmetika, 2. Aufl. (1989), S. 786).

Diese seit Jahrzehnten geübte Praxis weist jedoch den Nachteil auf, daß mit weiteren Rezepturbestandteilen Unverträglichkeiten auftreten können. Darüberhinaus ist bei der Applikation der anwendungsfertigen Färbemischung, d.h. bei der Mischung der Oxidationsfarbstoffvorprodukt-Zusammensetzung, die in der Regel eine wäßrige Lösung, Emulsion, Dispersion oder Gel darstellt, mit der Peroxid-Zusammensetzung ein erheblicher Peroxid-Überschuß erforderlich, um die antioxidierende Wirkung dieser Reduktionsmittel aufzuheben. Gleichwohl kann die Färbereaktion verzögert sein, was sich auf die Qualität der erhaltenden Färbung, insbesondere deren Intensität, auswirken kann.

Es wurde nunmehr überraschenderweise gefunden, daß sich dieses Problem dadurch lösen läßt, wenn man
a) als Oxidationsfarbstoffvorprodukte eine Kombination aus mindestens einem Tetraaminopyrimidin, insbesondere 2,4,5,6-Tetraaminopyrimidin, und/oder einem Hydroxytriaminopyrimidin, insbesondere 4-Hydroxy-2,5,6-triaminopyrimidin bzw. deren Salzen, und
b) mindestens einer Kupplersubstanz, ausgewählt aus Resorcin, 2-Methylresorcin, 4-Chlorresorcin, 2-Aminophenol, 3-Aminophenol, 5-Amino-2-methylphenol, 6-Amino-2-methylphenol und/oder α-Naphtol, 2,5-Diaminopyridin, 2,6-Diaminopyridin, 2-Dimethylamino-5-aminopyridin, 2-Amino-3-hydroxypyridin und/oder 3-Amino-2-methylamino-6-methoxypyridin bzw. deren Salzen, 1,3-Diaminobenzol, 2-Amino-4-hydroxyethylaminoanisol und/oder 2,4-Diaminophenoxyethanol bzw. deren Salzen,
c) Dihydroxyaceton und/oder Alloxan und/oder Methylglyoxal, und
d) Kaliumjodid einsetzt.

Wegen ihrer Verwandtschaft zu den Sonnenschutzpräparaten sollen hier auch die sogenannten Bräunungsmittel angeführt werden, Präparate, die ohne Sonneneinwirkung ein Hautbräunung herbeiführen. Die Wirksubstanz ist das Dihydroxyaceton, ein zu den Monosacchariden gehörendes Kohlehydrat ―C₃H₆O₃― Strukturformel 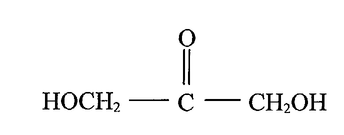 Dihydroxyaceton reagiert mit den Eiweißkörpern der Haut unter Bildung brauner Verbindungen. Die Wirkung beruht auf einer Reaktion des Ketozuckers mit freien Aminogruppen der Hautproteine. Die mit diesem Präparat gebräunte Haut ist nicht gegen Sonnenbrand geschützt. Günstige Hautbräunungseffekte werden durch Kombination aus Dihydroxyaceton und/oder Alloxan und/oder etwas Methylglyoxal bei pH 4 bis 10,5, insbesondere bei 5 bis 8, erzielt.
Die Färbung tritt nach 3 bis 5 Stunden ein und hält einige Tage an. Eine Kombination mit Walnußschalenextrakt sorgt für einen echten Braunton und gibt gleichzeitig einen Lichtschutzeffekt.
Nach Untersuchungen von Tronnier, Mayerus, Rapp und Schmitt ist die nicht-enzymatische Bräunung der Haut mit Dihydroxyaceton wesentlich von den Rezepturen abhängig und kann durch Vorreinigung der Haut, Belichtung und zweckmäßiger Nachbehandlung intensiviert werden. Während die Hauttemperatur bei diesem Versuch völlig unbeeinflußt blieb, zeigte die Wasserabgabe und Hydratation der Hornschicht deutliche Änderungen und zwar eine Verminderung der Feuchtigkeitsabgabe und wohl mehr lokal eine Abnahme der Hydratation unter der Belichtung, bevorzugt in den seborrhoischen Zonen.
Die Elastizität der Haut nimmt als Folge der Hornschichtverdickung ab, zeigt aber in der Dermatitis solaris infolge des Ödems eine Verstärkung. Eine Zunahme der Faltenbildung war in der kurzen zu übersehenden Versuchszeit nur andeutungsweise vorhanden. Siehe auch F. Greiter, "Künstliche Hautbräunungsmittel - Problematik und mögliches Modell", Parf. und Kosmetik. 55, Nr. 9/1974, 264-265.Bei Anwendung dieser Gemische mit Peroxiden, insbesondere Wasserstoffperoxid, werden nach kurzer Einwirkungszeit ausdrucksvolle, intensive Färbungen im Rotbereich erhalten, die durch Zusatz weiterer Kupplersubstanzen auch zu anderen Nuancen variierbar sind.

Die eingesetzten Tetraaminopyrimidine sind ebenso wie die Hydroxytriaminopyrimidine als Entwicklersubstanzen in Haarfärbemitteln an sich bekannt, letztere z.B. aus der EP-B 467 026, und bedürfen keiner näheren Erläuterung.

Sie können als freie Basen oder, sofern aus Löslichkeitsgründen erforderlich, auch als wasserlösliche Salze, insbesondere als Hydrochloride oder Sulfate, eingesetzt werden.

Die erfindungsgemäßen Haarfärbemittel können, neben den obengenannten essentiellen Bestandteilen weitere Oxidationsfarbstoffvorprodukte enthalten.
Beispiele hierfür sind 1-Methoxy-2-amino-4-(β-hydroxyethylamino)benzol, 2-Amino-N,N-diethylaminotoluol, 2-Amino-4-chlorphenol, 1,6-Dihydroxynaphthalin, 1,7-Dihydroxynaphthalin, 1-Methyl-2-hydroxy-4-aminobenzol, 5-Amino-2-methoxyphenol, 2-Methyl-5-hydroxyethylaminophenol, 4-Amino-3-methylphenol, bzw. deren wasserlöslichen Salze.
Damit soll jedoch der Zusatz weiterer Entwickler- und Kupplersubstanzen keineswegs ausgeschlossen sein.

Bei Anwendung dieser Zusammensetzungen auf Basis einer üblichen Grundlage werden, wie bereits erwähnt, nach der Oxidation mit Peroxid auch schon nach relativ kurzer Einwirkungszeit sehr ausdrucksvolle, intensive, dauerhafte Haarfärbungen vor allem im Rotbereich erhalten, die durch Zusatz entsprechender weiterer Kupplersubstanzen noch zu anderen Farbnuancen variiert werden können

Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.
Das bevorzugte Gewichtsverhältnis der genannten Entwicklersubstanzen zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.
Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,01 bis 5,0 %, vorzugsweise 0,05 bis 4 %, insbesondere 0,1 bis 3 Gew.-% der Gesamtzusammensetzung (ohne Oxidationsmittel), wobei sich die Angaben jeweils auf den Anteil an freier Base beziehen.
Der Anteil an Dihydroxyaceton und/oder Alloxan und/oder Methylglyoxal liegt vorzugsweise bei etwa 0,05 bis 5, vor allem 0,25 bis 2,5, insbesondere bei etwa 0,5 bis 2 Gew.-% des Färbemittels (ohne Oxidationsmittelzusammensetzung).
Das Kaliumjodid ist bekannt und bedarf deshalb keiner weiteren Erwähnung.
Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc..

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10 liegen

Die folgenden Beispiele dienen der Illustration der Erfindung.

| **Grundlage** | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Eiweißhydrolysat | 0,5 |
| Parfum | 0,4 |
| Ammoniak, 25%ig | 8,5 |
| Ammoniumchlorid | 0,5 |
| Panthenol | 0,8 |
| Kaliumjodid | 0,0001 bis 0,500 |
| Dihydroxyaceton und/oder Alloxan und/oder Methylglyoxal | 0,05 bis 5,00 |
| Wasser | ad 100,0 |

Die erfindungsgemäße Entwickler-Kuppler-Kombination wurde, jeweils unter entsprechender Verringerung des Wassergehalts, in diese Grundlage eingearbeitet. Die Ausfärbungen erfolgen jeweils an Woll-Läppchen und Strähnen aus gebleichtem Menschenhaar durch Aufbringung einer einen alkalischen pH-Wert aufweisenden Mischung aus Farbstoff-Vorprodukt und 6%-iger Wasserstoffperoxid-Lösung und zwanzigminütiger Einwirkung bei Zimmertemperatur, Auswaschen und Trocknen.

Es wurden die folgenden Färbungen erzielt:

| **Beispiele (in Gew.-%)** | | | | | | | | | | |
|---|---|---|---|---|---|---|---|---|---|---|
| **Nr.** | **1** | **1a** | **2** | **2a** | **3** | **3a** | **4** | **4a** | **5** | **5a** |
| **2,4,5,6-Tetraaminopyrimidinsulfat** | **2,20** | **2,20** | **2,20** | **2,20** | **2,20** | **2,20** | **2,20** | **2,20** | **2,20** | **2,20** |
| **Resorcin** | **1,00** | **1,00** | **1,00** | **1,00** | **1,00** | **1,00 -** | | **-** | **-** | **-** |
| **2-Methylresorcin** | **-** | **-** | **-** | **-** | **-** | **-** | **1,15** | **1,15** | **1,15** | **1,15** |
| **4-Chlorresorcin** | **-** | **-** | **-** | **-** | **-** | **-** | **-** | **-** | **-** | **-** |
| **Natriumsulfit** | **-** | | **1,00** | **1,00** | **1,00** | **1,00 -** | | **-** | **-** | **-** |
| **Ascorbinsäure** | **0,50 -** | | **-** | **-** | **1,50** | **1,50** | **1,00** | **1,00** | **1,20** | **1,20** |
| **Dihydroxyaceton** | **1,00** | **-** | **-** | **-** | **1,00** | **-** | **1,00** | **-** | **1,00** | **-** |
| **Kaliumjodid** | **0,005** | **-** | **0,005** | **-** | **0,005** | **-** | **0,005** | **-** | **0,001** | **-** |

| **Beispiele (in Gew.-%)** | | | | | | | | | | |
|---|---|---|---|---|---|---|---|---|---|---|
| **Nr.** | **6** | **6a** | **7** | **7a** | **8** | **8a** | **9** | **9a** | **10** | **10a** |
| **2,4,5,6-Tetraaminopyrimidinsulfat** | 2,20 | 2,20 | 2,20 | 2,20 | 2,20 | 2,20 | 2,20 | 2,20 | 2,20 | 2,20 |
| **Resorcin** | **-** | **-** | **-** | **-** | **-** | **-** | **-** | **-** | **-** | **-** |
| **2-Methylresorcin** | **1,15** | **1,15** | **1,15** | **1,15** | **-** | **-** | **-** | **-** | **-** | **-** |
| 4-Chlorresorcin | - | - | - | - | 1,32 | 1,32 | 1,32 | 1,32 | 1,32 | 1,32 |
| **Natriumsulfit** | **-** | **-** | **-** | **-** | **0,50** | **0,50** | **-** | **-** | **-** | **-** |
| **Ascorbinsäure** | **1,20** | **1,20** | **1,20** | **1,20 -** | | **-** | **1,50** | **1,50** | **1,50** | **1,50** |
| **Dihydroxyaceton** | **-** | **-** | **0,80** | **-** | **2,00** | **-** | **2,00 -** | | **-** | **-** |
| **Kaliumjodid** | **0,005** | **-** | **0,001** | **-** | **0,005** | **-** | **0,002** | **-** | **0,005** | **-** |

| **Beispiele (in Gew.-%)** | | | | | | | | | | |
|---|---|---|---|---|---|---|---|---|---|---|
| **Nr.** | **11** | **11a** | **12** | **12a** | **13** | **13a** | **14** | **14a** | **15** | **15a** |
| **4-Hydroxy-2,5,6-triamino- pyrimidinsulfat** | **2,20** | **2,20** | **2,20** | **2,20** | **2,20** | **2,20** | **2,20** | **2,20** | **2,20** | **2,20** |
| **Resorcin** | **1,00** | **1,00** | **1,00** | **1,00** | **1,00** | **1,00** | **-** | **-** | **-** | **-** |
| **2-Methylresorcin** | **-** | **-** | **-** | **-** | **-** | **-** | **1,15** | **1,15** | **1,15** | **1,15** |
| **4-Chlorresorcin** | **-** | **-** | **-** | **-** | **-** | **-** | **-** | **-** | **-** | **-** |
| **Natriumsulfit** | **0,50** | **0,50** | **-** | **-** | **-** | **-** | **-** | **-** | **1,50** | **1,50** |
| **Ascorbinsäure** | **-** | **-** | **0,90** | **0,90** | **1,00** | **1,00** | **-** | **-** | **-** | **-** |
| **Dihydroxyaceton** | **1,50** | **-** | **-** | **-** | **2,10** | **-** | **-** | **-** | **1,50** | **-** |
| **Kaliumjodid** | **0,0006** | **-** | **0,0006** | **-** | **0,0005** | **-** | **0,001** | **-** | **-** | **-** |

| **Beispiele (in Gew.-%)** | | | | | | | | | | |
|---|---|---|---|---|---|---|---|---|---|---|
| **Nr.** | **16** | **16a** | **17** | **17a** | **18** | **18a** | **19** | **19a** | **20** | **20a** |
| **4-Hydroxy-2,5,6-triamino- pyrimidinsulfat** | **2,20** | **2,20** | **2,20** | **2,20** | **2,20** | **2,20** | **2,20** | **2,20** | **2,20** | **2,20** |
| **Resorcin** | **-** | **-** | **-** | **-** | **-** | **-** | **-** | **-** | **-** | **-** |
| **2-Methylresorcin** | **1,15** | **1,15** | **1,15** | **1,15** | **-** | **-** | **-** | **-** | **-** | **-** |
| **4-Chlorresorcin** | **-** | **-** | **-** | **-** | **1,32** | **1,32** | **1,32** | **1,32** | **1,32** | **1,32** |
| **Natriumsulfit** | **-** | **-** | **1,50** | **1,50** | **1,20** | **1,20** | **2,50** | **2,50** | **0,80** | **0,80** |
| **Ascorbinsäure** | | | **0,50** | **0,50** | **-** | **-** | **1,50** | **1,50** | **-** | **-** |
| **Dihydroxyaceton** | **1,50** | **-** | **1,05** | **-** | **-** | **-** | **1,05** | **-** | **1,05** | **-** |
| **Kaliumjodid** | **-** | **-** | **0,05** | **-** | **0,004** | **-** | **0,05** | **-** | **0,02** | **-** |

In allen erfindungsgemäßen Zusammensetzungen wurde eine karminrote Färbung erhalten, die eine stärkere Farbintensität sowie einen ausgeprägteren Glanz als die nicht erfindungsgemäßen Zusammensetzungen aufwiesen.
Auch erfolgte der Farbaufzug schneller.

| **Beispiele (in Gew.-%)** | | | | | | | | | |
|---|---|---|---|---|---|---|---|---|---|
| **Nr.** | **24** | **24a** | **24b** | **25** | **25a** | **25b** | **26** | **26a** | **26b** |
| **2,4,5,6-Tetraaminopyrimidinsulfat** | **2,20** | **2,20** | **2,20** | **2,20** | **2,20** | **2,20** | **2,20** | **2,20** | **2,20** |
| **α-Naphthol** | **1,30** | **1,30** | **1,30** | **-** | **-** | **-** | **-** | **-** | **-** |
| **3-Aminophenol** | **-** | **-** | **-** | **1,00** | **1,00** | **1,00** | **-** | **-** | **-** |
| **5-Amino-2-methylphenol** | **-** | **-** | **-** | **-** | **-** | **-** | **1,15** | **1,15** | **1,15** |
| **Natriumsulfit** | **1,00** | **1,00** | **1,00** | **1,00** | **1,00** | **1,00** | **1,00** | **1,00** | **1,00** |
| **Methylglyoxal** | **-** | **-** | **0,50** | **-** | **0,50** | **0,50** | **-** | **-** | **-** |
| **Alloxan** | **-** | **0,50** | **-** | **-** | **0,50** | **-** | **-** | **0,70** | **0,50** |
| **Kaliumjodid** | **-** | **0,003** | **0,003** | **-** | **0,005** | **0,005** | **-** | **0,003** | **0,003** |
| **Färbung** | **Mattbraun** | | | **Violettstichiges Braun** | | | **Violettstichiges Blau** | | |

| **Beispiele (in Gew.-%)** | | | | | | | | | |
|---|---|---|---|---|---|---|---|---|---|
| **Nr.** | **24** | **24a** | **24b** | **25** | **25a** | **25b** | **26** | **26a** | **26b** |
| **4-Hydroxy-2,5,6-triaminopyrimidinsulfat** | 2,20 | 2,20 | 2,20 | 2,20 | 2,20 | 2,20 | 2,20 | 2,20 | 2,20 |
| **α-Naphthol** | **1,30** | **1,30** | **1,30** | **-** | **-** | **-** | **-** | **-** | **-** |
| **3-Aminophenol** | **-** | **-** | **-** | **1,00** | **1,00** | **1,00** | **-** | **-** | **-** |
| **5-Amino-2-methylphenol** | **-** | **-** | **-** | **-** | **-** | **-** | **1,15** | **1,15** | **1,15** |
| **Natriumsulfit** | **1,00** | **1,00** | **1,00** | **1,00** | **1,00** | **1,00** | **1,00** | **1,00** | **1,00** |
| **Methylglyoxal** | **-** | **0,80** | **0,50** | **-** | **-** | **0,50** | **-** | **1,00** | **0,50** |
| **Alloxan** | **-** | **0,50** | **-** | **-** | **-** | **-** | **-** | **0,50** | **-** |
| **Kaliumjodid** | **-** | **0,003** | **0,007** | **-** | **0,003** | **0,007** | **-** | **0,003** | **0,007** |
| **Färbung** | **Blauviolett** | | | **Braunviolett** | | | **Rotviolett** | | |

In allen Fällen wiesen überraschenderweise die erfindungsgemäßen Zusammensetzungen eine stärkere Farbintensität sowie einen ausgeprägteren Glanz auf.

| Beispiele (in Gew.-%) | | | | | | | | | |
|---|---|---|---|---|---|---|---|---|---|
| **Nr.** | **27** | **27a** | **27b** | **28** | **28a** | **28b** | **29** | **29a** | **29b** |
| **2,4,5,6-Tetraaminopyrimidinsulfat** | **2,20** | **2,20** | **2,20** | **2,20** | **2,20** | **2,20** | **2,20** | **2,20** | **2,20** |
| **2,6-Diaminopyridin** | **1,00** | **1,00** | **1,00** | **-** | **-** | **-** | **-** | **-** | **-** |
| **3-Amino-2-methylamino-6-methoxypyridindihydrochlorid** | **-** | **-** | **-** | **2,20** | **2,20** | **2,20** | **-** | **-** | **-** |
| **2-Amino-3-hydroxypyridin** | **-** | **-** | **-** | **-** | **-** | **-** | **1,00** | **1,00** | **1,00** |
| **Natriumsulfit** | **1,00** | **1,00** | **1,00** | **1,00** | **1,00** | **1,00** | **1,00** | **1,00** | **1,00** |
| **Methylglyoxal** | **-** | **-** | **0,50** | **0,50** | **-** | **0,50** | **-** | **-** | **-** |
| **Alloxan** | **-** | **0,50** | **-** | **0,50** | **-** | **-** | **-** | **0,70** | **0,50** |
| **Kaliumjodid** | **-** | **0,003** | **0,003** | **0,005** | **-** | **0,005 -** | **-** | **0,003** | **0,003** |
| **Färbung** | **Olivgrün** | | | **Olivgrün** | | | **Braunorange** | | |

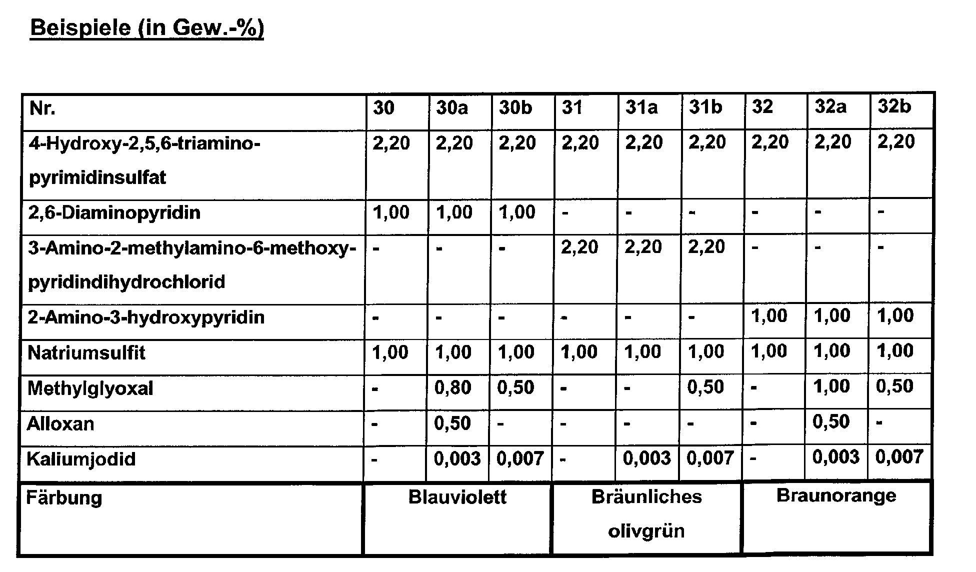

In allen Fällen wiesen überraschenderweise die erfindungsgemäßen Zusammensetzungen eine stärkere Farbintensität sowie einen ausgeprägteren Glanz auf.

| **Beispiele (in Gew.-%)** | | | | | | | | | |
|---|---|---|---|---|---|---|---|---|---|
| **Nr.** | **33** | **33a** | **33b** | **34** | **34a** | **34b** | **35** | **35a** | **35b** |
| **2,4,5,6-Tetraaminopyrimidinsulfat** | **1,08** | **1,08** | **1,08** | **1,08** | **1,08** | **1,08** | **1,08** | **1,08** | **1,08** |
| **1,3-Diaminobenzol** | **0,50** | **0,50** | **0,50** | **-** | **-** | **-** | **-** | **-** | **-** |
| **2-Amino-4-hydroxyethylamino- anisolsulfat** | **-** | **-** | **-** | **1,25** | **1,25** | **1,25** | **-** | **-** | **-** |
| **2,4-Diaminophenoxyethanol- hydrochlorid** | **-** | **-** | **-** | **-** | **-** | **-** | **1,10** | **1,10** | **1,10** |
| **Natriumsulfit** | **1,00** | **1,00** | **1,00** | **1,00** | **1,00** | **1,00** | **1,00** | **1,00** | **1,00** |
| **Methylglyoxal** | **-** | **-** | **0,50** | **-** | **0,50** | **0,50** | **-** | **-** | **-** |
| **Alloxan** | **-** | **0,50** | **-** | **-** | **0,50** | **-** | **-** | **0,70** | **0,50** |
| **Kaliumjodid** | **-** | **0,003** | **0,003** | **-** | **0,005** | **0,005** | **-** | **0,003** | **0,003** |
| **Färbung** | **Gelbgrün** | | | **Grünblau** | | | **Dunkeltürkis** | | |

| **Beispiele (in Gew.-%)** | | | | | | | | | |
|---|---|---|---|---|---|---|---|---|---|
| Nr. | 36 | 36a | 36b | 37 | 37a | 37b | 38 | 38a | 38b |
| **4-Hydroxy-2,5,6-triaminopyrimidinsulfat** | **1,08** | **1,08** | **1,08** | **1,08** | **1,08** | **1,08** | **1,08** | **1,08** | **1,08** |
| **1,3-Diaminobenzol** | **0,50** | **0,50** | **0,50** | **-** | **-** | **-** | **-** | **-** | **-** |
| **2-Amino-4-hydroxyethylaminoanisolsulfat** | **-** | **-** | **-** | **1,25** | **1,25** | **1,25** | **-** | **-** | **-** |
| **2,4-Diaminophenoxyethanolhydrochlorid** | **-** | **-** | **-** | **-** | **-** | **-** | **1,10** | **1,10** | **1,10** |
| **Natriumsulfit** | 1,00 | 1,00 | 1,00 | 1,00 | 1,00 | 1,00 | 1,00 | 1,00 | 1,00 |
| **Methylglyoxal** | **-** | **0,80** | **0,50** | **-** | **-** | **0,50** | **-** | **1,00** | **0,50** |
| **Alloxan** | **-** | **0,50** | **-** | **-** | **-** | **-** | **-** | **0,50** | **-** |
| **Kaliumjodid** | **-** | **0,003** | **0,007** | **-** | **0,003** | **0,007** | **-** | **0,003** | **0,007** |
| **Färbung** | **Silberbraun** | | | **Dunkelblauviolett** | | | **Hellblauviolett** | | |

Überraschenderweise wiesen die erfindungsgemäßen Zusammensetzungen durchgängig eine stärkere Farbintensität sowie einen ausgeprägteren Glanz auf.

## Patentansprüche

1. Wäßrige Haarfärbemittelzusammensetzung auf Basis eines Oxidationsfarbstoffvorprodukt-Systems, enthaltend
a) mindestens ein Tetraaminopyrimidin und/oder Triaminohydroxypyrimidin bzw. deren wasserlösliche Salze, und
b) mindestens eine Kupplersubstanz, ausgewählt aus Resorcin, 2-Methylresorcin, 4-Chlorresorcin und/oder 2-Aminophenol, 3-Aminophenol, 5-Amino-2-methylphenol, 6-Amino-2-methylphenol, α-Naphtol und/oder 2,5-Diaminopyridin, 2,6-Diaminopyridin, 2-Dimethylamino-5-aminopyridin, 2-Amino-3-hydroxypyridin, 3-Amino-2-methylamino-6-methoxypyridin, bzw. deren Salzen und/oder 1,3-Diaminobenzol, 2-Amino-4-hydroxyethylaminoanisol und/oder 2,4-Diaminophenoxyethanol bzw. deren Salzen,
c) Dihydroxyaceton, Alloxan und Methylglyoxal und
d) Kaliumjodid.

2. Haarfärbemittel nach Anspruch 1, enthaltend als Entwicklersubstanz 2,4,5,6-Tetraaminopyrimidin und/oder ein wasserlösliches Salz desselben.

3. Haarfärbemittel nach Anspruch 1, enthaltend 4-Hydroxy-2,5,6-triaminopyrimidin und/oder ein wasserlösliches Salz desselben.

4. Verfahren zum Färben von menschlichen Haaren, wobei eine wäßrige Oxidationsfarbstoffvorprodukt-Zusammensetzung, enthaltend
a) mindestens ein Tetraaminopyrimidin und/oder Triaminohydroxypyrimidin bzw. deren wasserlösliche Salze, und
b) mindestens eine Kupplersubstanz, ausgewählt aus Resorcin, 2-Methylresorcin, 4-Chlorresorcin und/oder 2-Aminophenol, 3-Aminophenol, 5-Amino-2-methylphenol, 6-Amino-2-methylphenol, α-Naphtol und/oder 2,5-Diaminopyridin, 2,6-Diaminopyridin, 2-Dimethylamino-5-aminopyridin, 2-Amino-3-hydroxypyridin, 3-Amino-2-methylamino-6-methoxypyridin, bzw. deren Salzen und/oder 1,3-Diaminobenzol, 2-Amino-4-hydroxyethylaminoanisol und/oder 2,4-Diaminophenoxyethanol bzw. deren Salzen,
c) Dihydroxyaceton, Alloxan und Methylglyoxal und
d) Kaliumjodid,
mit einer wäßrigen Peroxid-Zusammensetzung gemischt, auf das Haar aufgebracht und nach erfolgter Einwirkung aus dem Haar ausgespült wird.
